# EUROPÄISCHE PATENTSCHRIFT

(11) **EP 2 082 696 B1**
(45) Veröffentlichungstag und Bekanntmachung des Hinweises auf die Patenterteilung: **28.03.2012**
(21) Anmeldenummer: 09000897.0
(22) Anmeldetag: 23.01.2009
(51) Int. Cl.: A61B 17/50, A61B 17/54, A61B 19/00, A61B 17/32, A61B 17/00

(54) **Abrasionsvorrichtung**
Abrasion device
Dispositif d'abrasion

(30) Priorität: 24.01.2008 DE 102008005940
(43) Veröffentlichungstag der Anmeldung: 29.07.2009
(73) Patentinhaber: La Fontaine, Helmut, 29604 Marbella Málaga (ES)
(72) Erfinder: La Fontaine, Helmut, 29604 Marbella Málaga (ES)
(74) Vertreter: Geitz Truckenmüller Lucht

(56) Entgegenhaltungen:
- WO-A-2006/002489
- WO-A-2007/080596
- FR-A- 2 846 221
- US-A- 4 494 555
- US-A1- 2004 030 325
- US-A1- 2004 127 914

## Beschreibung

Die vorliegende Erfindung betrifft eine Abrasionsvorrichtung mit einem Griffgehäuse und einer eine Vielzahl von Erhebungen aufweisenden Abrasionseinheit zur Hautbehandlung, wobei das Griffgehäuse einen elektrischen Antrieb zur Kraftübertragung auf die Abrasionseinheit aufweist.

Eine solche Abrasionsvorrichtung gemäß dem Oberbegriff des Anspruchs 1 ist bereits aus der WO 2007/080596 A2 vorbekannt. Hierbei handelt es sich um eine Vorrichtung zum abrasiven Abtragen der obersten Hautschichten. Die Vorrichtung besteht aus einer im wesentlichen in einer Ebene auf Kreis- oder Ellipsenbahnen schwingenden Nadelplatte.

Ebenfalls ist aus der EP 1 764 010 A1 ein Hautbearbeitungswerkzeug bekannt, welches als Kombinationswerkzeug ausgelegt ist. Diese dient zur großflächigen Selbstbehandlung der Hautoberfläche mit einer Applikationseinrichtung, die einen Bearbeitungskorpus und integriertem Lang- und Kurzhaarschneider aufweist. Mithilfe der Applikationseinrichtung kann bei der Behandlung ein Wirkstoff aus einem Speicher auf die Haut abgegeben werden.

Dokument FR-A-2846221 offenbart eine Abrasionsvorrichtung mit einem Griffgehäuse und einer eine Vielzahl von Erhebungen aufweisenden Abrasionseinheit zur Probenentnahme, wobei die Abrasionseinheit gegenüber dem Griffgehäuse federnd gelagert ist und von einer bezüglich des Griffgehäuses feststehenden Schutzhülse derart umgriffen ist, dass die Oberkante der Schutzhülse im unbelasteten Zustand hinter einer Abrasionsfläche der Abrasionseinheit liegt und die federnde Lagerung der Abrasionsfläche bei zu starkem Druck auf die Abrasionseinheit ein Ausweichen derselben bis hinter die Oberkante der Schutzhülse erlaubt. Das Griffgehäuse kann auch einen elektrischen Antrieb zur Kraftübertragung auf die Abrasionseinheit aufweisen.

Ferner kennt der Stand der Technik Vorrichtungen, mithilfe derer Partikel unter Verwendung eines von einer Vakuumpumpe erzeugten Unterdrucks auf die Hautoberfläche geschleudert und sogleich wieder abgesaugt werden. Dies erlaubt jedoch erfahrungsgemäß kein definiertes, gleichmäßiges Abtragen der oberen Hautschichten, weshalb an dieser Stelle stets eine Verletzungsgefahr besteht, z.B. durch zu langes Verweilen an einem Ort, da die Hautsensoren ein zu starkes Abtragen physiologisch nicht oder lediglich zu spät, nämlich erst nach Eintreten einer Hautverletzung, registrieren. Auch ist kein punktuelles Arbeiten auf kleinen Hautarealen, wie z.B. um Papeln herum möglich.

Auch die vorher genannten Vorrichtungen weisen mehrere Nachteile auf. So besitzen die genannten Vorrichtungen eine nicht auf die zu behandelnde Hautfläche abgestimmte Plattengeometrie, welche vor allem bei in der Behandlungsfläche liegenden lokalen Hautveränderungen, wie z.B. Papeln, Pusteln, Leberflecken, wichtig ist. Darüber hinaus ist auch die Eindringtiefe der Nadeln in die Haut nicht begrenzt, was je nach Anwendungsfall ebenfalls zu Verletzungen oder zumindest Reizungen des Hautorgans führt.

Vor diesem Hintergrund liegt der Erfindung die Aufgabe zu Grunde, eine Abrasionsvorrichtung zu schaffen, welche Verletzungen und unerwünschte Reizungen der Haut, etwa Rötungen (Erytheme), vermeidet.

Gelöst wird diese Aufgabe durch eine Abrasionsvorrichtung gemäß den Merkmalen des Hauptanspruchs. Weitere sinnvolle Ausgestaltungen können den Unteransprüchen entnommen werden.

Erfindungsgemäß wird die Abrasionsvorrichtung mit einem Griffgehäuse und einer Abrasionseinheit ausgeführt. Die Abrasionseinheit weist eine zur Anwendung auf der Haut vorgesehene Abrasionsfläche auf, welcher Erhebungen zur mechanischen Bearbeitung der Haut zugeordnet sind. Um einen zu großen Druck der Abrasionseinheit gegen die behandelte Haut zu vermeiden, ist die Abrasionseinheit gegenüber dem Griffgehäuse federnd gelagert, nämlich derart, dass die Abrasionseinheit bei einem zu großen Anpressdruck von der Haut weg ausweicht. Dabei federt mit Vorteil auch die Antriebswelle teilweise mit, indem eine federnde Lagerung in der Welle vorgesehen ist, die ein Zusammenschieben der Welle in ihrer Längsrichtung ermöglicht. Alternativ kann nicht zur Erfindung gehörend, auch der ganze Antrieb, welcher zur Übertragung einer Rotationsbewegung auf die Abrasionseinheit vorgesehen ist, mit der Abrasionseinheit mitfedern, so dass eine starre Verbindung zwischen Abrasionseinheit und Antrieb ausgebildet sein kann.

Um die Abrasionseinheit ist ferner eine Schutzhülse angeordnet, deren Oberkante im unbelasteten Zustand hinter der Abrasionsfläche liegt. Bei einem zu starken Druck auf die Abrasionseinheit weicht diese jedoch aus, im Grenzfall bis hinter die Oberkante der Schutzhülse, so das die Abrasionsfläche außer Eingriff der Haut gerät. Dadurch ist eine Anwendung nur mit einem je nach Behandlungsziel definierbaren Höchstmaß an Druck vornehmbar, welche durch die Federkonstante und die Höhe der Schutzhülse vorgegeben ist. Es ist hierbei zu erwähnen, dass die Feder austauschbar ist, so dass eine Anpassung der Federkonstante auf die jeweils zu behandelnde Haut erfolgen kann.

Bei unterschiedlichen Anwendungen - etwa bei unterschiedlichen Hauttypen oder verschiedenen Körperstellen - kann es jedoch auch sinnvoll sein, wenn unterschiedliche Druckbegrenzungen vorgenommen werden können. Hierzu kann die Schutzhülse in Federrichtung der Abrasionseinheit verschieblich sein, so dass die Oberkante je nach Anwendung vor- oder zurückverschoben werden kann. Hierbei kann eine Verrastung oder Verklemmung in der gewählten Position vorgenommen werden. Zudem können Hülsen verschiedener Formen verwendet werden, etwa eine Hülse mit einer die Abrasionsfläche umgebenden Randfläche für eine Behandlung großflächiger Hautbereiche oder eine Spitz zulaufende Hülse für schwer zugängliche Bereiche.

Die Erhebungen der Abrasionseinheit sind im Wesentlichen auf der Abrasionsfläche angeordnet, welche sich auf einer Stirnseite der Abrasionseinheit befindet. Ihre Wirkungsrichtung ist hierbei so gewählt, dass sie möglichst senkrecht auf der Haut angewendet werden sollen. Nachdem jedoch im Rahmen der Anwendung der Abrasionsvorrichtung eine Verschiebung über die Haut hinweg erfolgen soll, kann es zu einer Beaufschlagung der Erhebungen von einer seitlichen Richtung kommen, so dass hier ebenfalls die Möglichkeit von unerwünschter Reizungen besteht. Um dies zu vermeiden sind die Erhebungen im Bereich der Außenkanten der Abrasionsfläche derart anwendungsspezifisch abgerundet, dass eine seitliche Beaufschlagung der Erhebungen von den genannten Rundungen aufgefangen wird.

Im Bereich der abrasiven Behandlung der Haut ist, wie in allen medizinischen Anwendungen, die Hygiene ein überaus wichtiger Aspekt. Deshalb ist die Abrasionseinheit mit der Abrasionsvorrichtung lösbar verbunden, etwa mittels eines Stempels, der in eine Clipverbindung eingreift. Eine solche Verbindung erlaubt einerseits die einfache Abtrennung der Abrasionseinheit von dem Griffgehäuse, um diese zu reinigen und zu sterilisieren und eine Austauscheinheit einzusetzen. Andererseits können auf diese Art und Weise Abrasionseinheiten verschiedener Form und Auflösung mit einem Griffgehäuse eingesetzt werden.

Ebenfalls aus hygienischen Überlegungen heraus kann die Clipverbindung als Einmalverschluss ausgestaltet sein. Dann ist dem Stempel der Abrasionseinheit ein Verschlussteil mit Mitnehmern für die Übertragung der Rotationsbewegung zugeordnet, wobei die Mitnehmer nur über eine Sollbruchstelle mit dem Stempel verbunden sind. Bei der Entnahme der Abrasionseinheit aus der Vorrichtung brechen die Sollbruchstellen auf und die Abrasionseinheit kann in der Vorrichtung nicht mehr bestimmungsgemäß wieder eingesetzt werden, weil ohne die Mitnehmer eine Rotationsbewegung nicht mehr möglich ist. Somit ist die Abrasionseinheit als Einweg-Element ausgeführt, welches nach einer Anwendung entsorgt wird. Eine erneute Sterilisation kann entfallen und es muss hier nicht das Risiko einer unzureichenden Sterilisation eingegangen werden. Das neu einzusetzende Element wird direkt aus einer sterilen Verpackung entnommen.

Die Abrasionseinheit kann entweder dadurch hergestellt sein, dass einer blanken Einheit Erhebungen zugeordnet werden, etwa eine Abrasionsfläche angefügt wird. Alternativ kann die Abrasionseinheit aber auch einstückig mittels Spritzguss oder Feinspritzguss hergestellt sein, wobei die Erhebungen in diesem Fall gleich an die Abrasionseinheit angeformt sind. Sowohl die Erhebungen, das zugrunde liegende Bett, als auch eine einstückig hergestellte Abrasionseinheit können entweder aus Kunststoff oder aus Metall hergestellt sein.

Die Erhebungen als solche können verschiedene Formen aufweisen. Unter anderem können die Erhebungen tetraeder- oder pyramidenförmig sein. Allgemein ist es möglich, eine beliebige regelmäßige Grundfläche der Erhebungen vorzusehen, sofern eine einzelne oder mehrere Spitzen oder Kronen im Kontaktbereich mit der Haut entstehen, die Erhebungen also im Wesentlichen kegelförmig ausgebildet sind. Eine weitere Möglichkeit besteht darin, die Erhebungen hobelförmig, gegebenenfalls mit Hinterschneidungen, vorzusehen, um den Hautabtrag zusätzlich zu verbessern.

Vorzugsweise ist die Höhe der Erhebungen zwischen 50 und 1500 µm zu wählen.

Die Abrasionsfläche rotiert um eine Drehachse mit einer Frequenz zwischen zumindest näherungsweise 5 und 100 Hz. Hierbei ist es besonders wünschenswert, wenn diese Frequenz einstellbar, vorzugsweise stufenlos einstellbar ausgelegt ist.

Die Abrasionsfläche kann, je nach Ausprägung und Anwendung etwa kreisförmig, viereckig oder dreieckig sein.

Die vorstehend beschriebene Erfindung wird nachfolgend anhand eines Ausführungsbeispiel näher erläutert.

Es zeigen:
- Figur 1: eine Abrasionsvorrichtung in einer Schnittdarstellung unter teilweiser Weglassung des Griffgehäuses,
- Figur 2: eine Abrasionseinheit zur Verwendung mit der Abrasionsvorrichtung aus Figur 1, und
- Figuren 3a-f: jeweils eine Abrasionseinheit mit einer speziellen Ausgestaltung der Erhebun- gen.

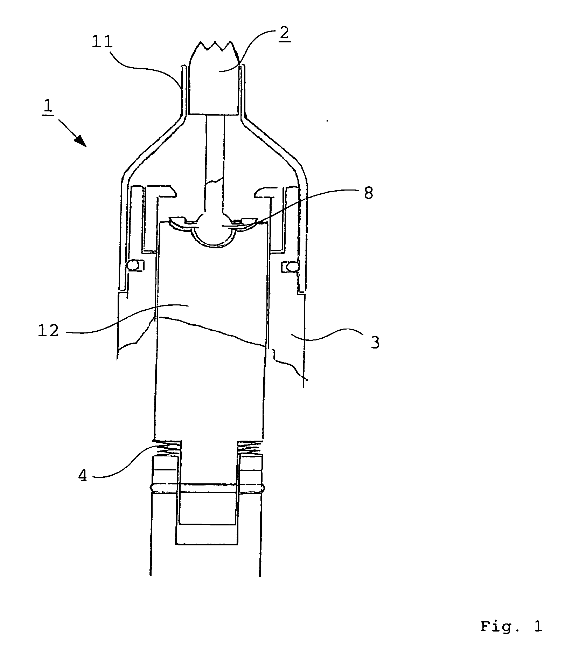

Figur 1 zeigt eine Abrasionsvorrichtung 1, welche im Wesentlichen aus einem Griffgehäuse 3 und einer Abrasionseinheit 2 hergestellt ist. Das Griffgehäuse 3 umfasst ferner einen elektrischen Antrieb, dessen Antriebswelle 12 mit der Abrasionseinheit 2 über eine Clipverbindung 8 wirkverbunden ist. Der elektrische Antrieb versetzt die Abrasionseinheit 2 in eine Rotationsbewegung im Bereich von 5 bis 100 Hz. Die derart rotierende Abrasionseinheit 2 wird sodann mittels des Griffgehäuses 3 in Eingriff mit der zu behandelnden Haut gebracht, so dass durch die Rotation der Abrasionseinheit 2 die oberste Hautschicht abgetragen wird. Dies muss mit einem geeigneten Druck auf die Haut erfolgen, da bei einem zu großen Druck eine Gefahr von Verletzungen besteht, weil gegebenenfalls zuviel Haut abgetragen wird. Dies wird jedoch vermieden, indem die Abrasionsvorrichtung mittels einer Federung 4 innerhalb des Griffgehäuses 3 gegenüber diesem federnd gelagert ist, so dass bei zu starkem Druck die Abrasionseinheit 2 in das Griffgehäuse 3 einrückt. Hierbei ist der elektrische Antrieb über eine federnde Antriebswelle 12 mit der Abrasionseinheit 2 wirkverbunden, so dass eine kraftschlüssige Verbindung zwischen Abrasionseinheit 2 und Antrieb geschaffen ist.

Um die Einrücktiefe in geeigneter Weise beeinflussen zu können, ist im Bereich des Durchtritts der Abrasionseinheit 2 durch die stirnseitige Gehäusewand eine Schutzhülse 11 vorgesehen, hinter deren Oberkante die Abrasionseinheit einrücken kann. Die Schutzhülse ist in ihrer Höhe verschiebbar und verrastbar, so dass die Einrücktiefe über die Position der Schutzhülse definierbar wird. Die Schutzhülse 11 kann auch ganz entfernt werden, so dass eine Sterilisierung vorgenommen oder eine neue, sterile Schutzhülse 11 eingesetzt werden kann.

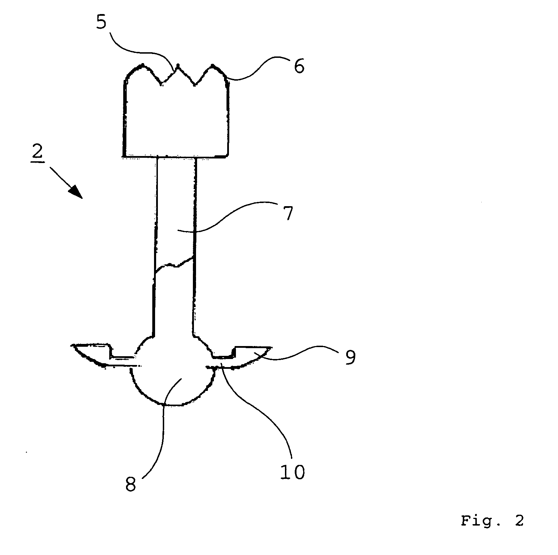

Figur 2 zeigt eine Abrasionseinheit 2 zur Verwendung mit der Abrasionsvorrichtung 1, welche im Wesentlichen aus einer Abrasionsfläche 5, einem Stempel 7 und einer Clipverbindung hergestellt ist. Die Abrasionsfläche weist in ihrem Randbereich eine Abrundung 6 auf, welche sicherstellen soll, dass eine Verletzung beim seitlichen Verschieben der Abrasionseinheit über die Haut nicht auftreten kann. Grundsätzlich wirken die eine Abrasionsfläche 5 bildenden Erhebungen hauptsächlich senkrecht zur Haut. Durch die Abrundungen erfolgt eine seitliche Wirkung bei der Verschiebung nicht, so dass auch hier eine kontrollierte Anwendung möglich ist. Durch den Einsatz verschiedener Abrasionseinheiten 2 mit verschiedenen Abrundungsgraden kann eine anwendungsspezifische Anpassung erfolgen.

Ferner ist die Abrasionsfläche in unterschiedlichen Größen einsetzbar, je nach gewünschter Anwendung. Dabei können bei kleineren Abrasionsflächen 5 Hautunreinheiten, wie beispielsweise Pickel, aber auch Hautveränderungen wie Leberflecke, besser umgangen werden.

Die Abrasionseinheit ist durch eine Clipverbindung 8 mit der Abrasionsvorrichtung 1 verbindbar, wobei die Clipverbindung 8 Mitnehmer 9 zur Übertragung der Rotationsbewegung vom elektrischen Antrieb auf die Abrasionseinheit 2 aufweist. Diese Mitnehmer 9 sind lediglich über Sollbruchstellen 10 mit der Clipverbindung 8 verbunden, so dass sie bei einer Entnahme brechen, mit der Folge, dass die Abrasionseinheit 2 nicht wiederverwendbar ist. Dies hat den Vorteil, dass eine Sterilisierung entfallen kann und trotzdem im Hinblick auf die geforderte Hygiene eine zufrieden stellende Lösung geboten wird.

Die Abrasionseinheit kann einstückig durch Spritzguss oder Feinspritzguss oder auch mehrstückig hergestellt sein.

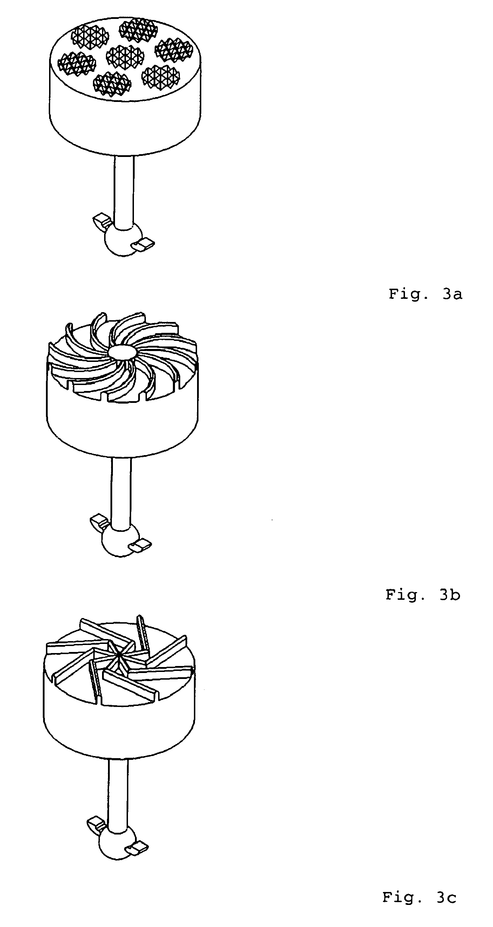

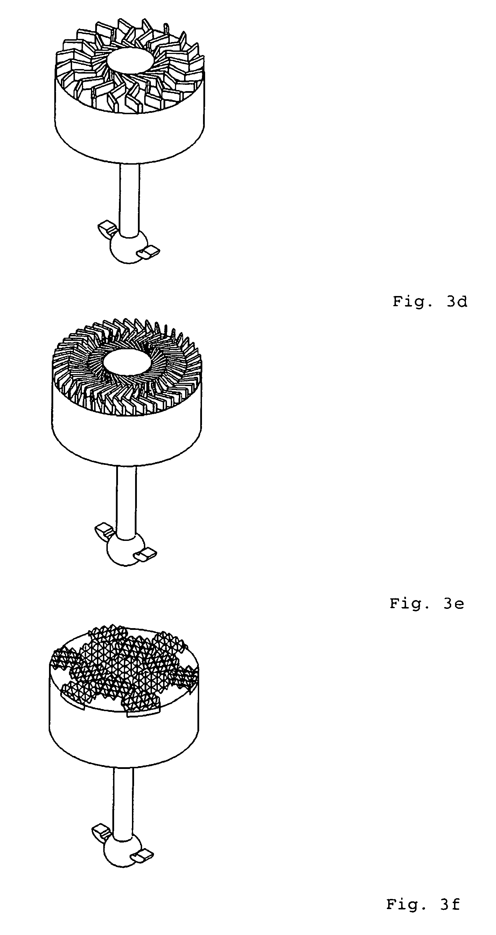

Figuren 3a-f zeigen eine Reihe verschiedener Ausgestaltungen der Abrasionseinheit, welcher der speziellen Anwendung angepasste Erhebungen zugeordnet sein können. So zeigt Figur 3a eine Anordnung mit einer Mehrzahl kreisförmiger Flächenabschnitte, und Figur 3f eine Anordnung mit sternförmig angeordneten Flächenabschnitten, welche jeweils pyramidenförmige Erhebungen aufweisen. Figuren 3b-e zeigen Abrasionseinheiten mit verschiedenartig wirbelförmigen Erhebungen, welche senkrecht oder schräg angestellt sein können und so eine besonders effektive Abrasion ermöglichen.

Vorstehend ist somit eine Abrasionsvorrichtung beschrieben, welche eine definierte Abrasion der Haut dadurch ermöglicht, dass bei zu hohem Anpressdruck auf die Haut ein Ausweichen der Abrasionseinheit durch Einrücken erfolgt, wodurch die Abrasionsfläche der Anordnung außer Eingriff der Haut gerät.

### BEZUGSZEICHENLISTE

- 1: Abrasionsvorrichtung
- 2: Abrasionseinheit
- 3: Griffgehäuse
- 4: Federung
- 5: Abrasionsfläche
- 6: Abrundung
- 7: Stempel
- 8: Clipverbindung
- 9: Mitnehmer
- 10: Sollbruchstelle
- 11: Schutzhülse
- 12: Antriebswelle

## Patentansprüche

1. Abrasionsvorrichtung mit einem Griffgehäuse (3) und einer eine Vielzahl von Erhebungen aufweisenden Abrasionseinheit (2) zur Hautbehandlung, wobei das Griffgehäuse (3) einen elektrischen Antrieb zur Kraftübertragung auf die Abrasionseinheit (2) aufweist, **dadurch gekennzeichnet, dass** die Abrasionseinheit (2) gegenüber dem Griffgehäuse (3) federnd gelagert und von einer bezüglich des Griffgehäuses (3) feststehenden Schutzhülse (11) derart umgriffen ist, dass die Oberkante der Schutzhülse (11) im unbelasteten Zustand hinter einer Abrasionsfläche (5) der Abrasionseinheit (2) liegt und die federnde Lagerung der Abrasionsfläche (5) bei zu starkem Druck auf die Abrasionseinheit (2) ein Ausweichen derselben bis hinter die Oberkante der Schutzhülse (11) erlaubt, wobei die Abrasionseinheit (2) über eine federnde Antriebswelle federnd gelagert ist, mittels dessen die Abrasionseinheit (2) im unbelasteten Zustand in eine Rotationsbewegung versetzbar ist.

2. Abrasionsvorrichtung gemäß Anspruch 1, **dadurch gekennzeichnet, dass** die Schutzhülse (11) in Federrichtung der Abrasionseinheit (2) verschieblich, jedoch verrastbar oder verklemmbar, ist.

3. Abrasionsvorrichtung gemäß einem der Ansprüche 1 oder 2, **dadurch gekennzeichnet, dass** die Erhebungen der Abrasionseinheit (2) auf der Abrasionsfläche (5) angeordnet sind, wobei die Erhebungen im Bereich der Außenkante der Abrasionsfläche (5) abgerundet sind.

4. Abrasionsvorrichtung gemäß einem der vorhergehenden Ansprüche, **dadurch gekennzeichnet, dass** die Abrasionseinheit (2) mit dem Antrieb, vorzugsweise über eine Clipverbindung (8), lösbar verbunden ist.

5. Abrasionsvorrichtung gemäß Anspruch 4, **dadurch gekennzeichnet, dass** die Clipverbindung (8) seitens der Abrasionseinheit (2) ein Einmalverschluss ist.

6. Abrasionsvorrichtung gemäß Anspruch 5, **dadurch gekennzeichnet, dass** der Einmalverschluss wenigstens eine Sollbruchstelle (10) aufweist, welche im Falle der Entnahme der Abrasionseinheit (2) aus der Clipverbindung (8) bricht.

7. Abrasionsvorrichtung gemäß einem der vorhergehenden Ansprüche, **dadurch gekennzeichnet, dass** die Abrasionseinheit (2) mit den Erhebungen und dem Einmalverschluss einstückig, vorzugsweise mittels Spritzguss oder Feinspritzguss, hergestellt ist.

8. Abrasionsvorrichtung gemäß einem der vorhergehenden Ansprüche, **dadurch gekennzeichnet, dass** die Erhebungen tetraeder-, pyramidenförmig oder zumindest weitgehend kegelförmig ausgebildet sind.

9. Abrasionsvorrichtung gemäß einem der vorhergehenden Ansprüche, **dadurch gekennzeichnet, dass** die Höhe der Erhebungen zwischen 50 und 1500 µm beträgt.

10. Abrasionsvorrichtung gemäß einem der vorhergehenden Ansprüche, **dadurch gekennzeichnet, dass** die Rotation der Abrasionseinheit (2) mit einer Frequenz von 50 bis 200 Hz erfolgt.

11. Abrasionsvorrichtung gemäß einem der vorhergehenden Ansprüche, **dadurch gekennzeichnet, dass** die Rotationsfrequenz der Abrasionseinheit (2), vorzugsweise stufenlos, einstellbar ist.

12. Abrasionsvorrichtung gemäß einem der Ansprüche 3 bis 12, **dadurch gekennzeichnet, dass** die Abrasionsfläche (5) kreisförmig ist und einen Flächendurchmesser zwischen 2 und 100 mm aufweist.

13. Abrasionsvorrichtung gemäß einem der Ansprüche 3 bis 12, **dadurch gekennzeichnet, dass** die Abrasionsfläche (5) dreieckig oder viereckig ist.

14. Abrasionsvorrichtung gemäß einem der vorhergehenden Ansprüche, **dadurch gekennzeichnet, dass** die Schutzhülse 11 abnehmbar ist.

## Claims

1. An abrasion device, comprising a grip housing (3) and an abrasion unit (2) for skin treatment having a plurality of elevations, with the grip housing (3) having an electric drive for power transmission onto the abrasion unit (2), **characterized in that** the abrasion unit (2) is resiliently supported in relation to the grip housing (3) and is encompassed by a protective sleeve (11) fixed in relation to the grip housing (3) in such a way that the upper edge of the protective sleeve (11) lies in the non-loaded state behind an abrasion surface (5) of the abrasion unit (2) and the resilient bearing of the abrasion surface (5), during excessive pressure on the abrasion unit (2), allows a yielding of said abrasion surface up to and behind the upper edge of the protective sleeve (11), with the abrasion unit (2) being resiliently mounted by means of a resilient drive shaft, by means of which the abrasion unit (2) can be made to rotate in the unloaded state.

2. An abrasion device according to claim 1, **characterized in that** the protective sleeve (11) is displaceable in the direction of the spring of the abrasion unit (2), but lockable or clampable.

3. An abrasion device according to one of the claims 1 or 2, **characterized in that** the elevations of the abrasion unit (2) are arranged on the abrasion surface (5), with the elevations being rounded off in the region of the outside edge of the abrasion surface (5).

4. An abrasion device according to one of the preceding claims, **characterized in that** the abrasion unit (2) is detachably connected with the drive, preferably by way of a clip connection (8).

5. An abrasion device according to claim 4, **characterized in that** the clip connection (8) is a one-time lock on the part of the abrasion unit (2).

6. An abrasion device according to claim 5, **characterized in that** the one-time lock has at least one predetermined breaking point, which will break in the case of the removal of the abrasion unit (2) from the clip connection (8).

7. An abrasion device according to one of the preceding claims, **characterized in that** the abrasion unit (2) with the elevations and the one-time lock is produced integrally, preferably by means of injection molding or fine injection molding.

8. An abrasion device according to one of the preceding claims, **characterized in that** the elevations are arranged in the manner of a tetraeder, pyramid or at least substantially in the shape of a cone.

9. An abrasion device according to one of the preceding claims, **characterized in that** the height of the elevations is between 50 and 1500 µm.

10. An abrasion device according to one of the preceding claims, **characterized in that** the rotation of the abrasion unit (2) occurs with a frequency of 50 to 200 Hz.

11. An abrasion device according to one of the preceding claims, **characterized in that** the rotation frequency of the abrasion unit (2) is adjustable, preferably in a continuously variable manner.

12. An abrasion device according to one of the claims 3 to 12, **characterized in that** the abrasion surface (5) is circular and has a surface diameter of between 2 and 100 mm.

13. An abrasion device according to one of the claims 3 to 12, **characterized in that** the abrasion surface (5) is triangular or quadrangular.

14. An abrasion device according to one of the preceding claims, **characterized in that** the protective sleeve (11) is removable.

## Revendications

1. Dispositif d'abrasion avec un boîtier formant poignée (3) et une unité d'abrasion (2) pour le traitement de la peau présentant de multiples saillies, dans lequel le boîtier formant poignée (3) présente un entraînement électrique pour la transmission de force à l'unité d'abrasion (2), **caractérisé en ce que** l'unité d'abrasion (2) est supportée de façon élastique par rapport au boîtier formant poignée (3) et entourée par un manchon de protection (11) fixe par rapport au boîtier formant poignée (3), de telle façon que le bord supérieur du manchon de protection (11) se trouve, dans l'état sans charge, en arrière d'une surface d'abrasion (5) de l'unité d'abrasion (2) et que le support élastique de la surface d'abrasion (5) permette, en cas de pression excessive sur l'unité d'abrasion (2), le recul de celle-ci en arrière du bord supérieur du manchon de protection (11), l'unité d'abrasion (2) étant supportée de façon élastique sur un arbre d'entraînement élastique au moyen duquel l'unité d'abrasion (2) dans l'état sans charge peut être entraînée dans un mouvement de rotation.

2. Dispositif d'abrasion selon la revendication 1, **caractérisé en ce que** le manchon de protection (11) est mobile dans le sens d'élasticité de l'unité d'abrasion (2) mais peut être emboîté ou serré.

3. Dispositif d'abrasion selon l'une des revendications 1 ou 2, **caractérisé en ce que** les saillies de l'unité d'abrasion (2) sont disposées sur la surface d'abrasion (5), les saillies étant arrondies au niveau du bord extérieur de la surface d'abrasion (5).

4. Dispositif d'abrasion selon l'une des revendications précédentes, **caractérisé en ce que** l'unité d'abrasion (2) est reliée à l'entraînement de façon amovible, de préférence par un assemblage clipsé (8).

5. Dispositif d'abrasion selon la revendication 4, **caractérisé en ce que** l'assemblage clipsé (8) est un verrouillage à usage unique du côté de l'unité d'abrasion (2).

6. Dispositif d'abrasion selon la revendication 5, **caractérisé en ce que** le verrouillage à usage unique présente au moins un point de rupture nominal (10) qui se détache de l'assemblage clipsé (8) en cas de retrait de l'unité d'abrasion (2).

7. Dispositif d'abrasion selon l'une des revendications précédentes, **caractérisé en ce que** l'unité d'abrasion (2) est fabriquée d'un seul tenant avec les saillies et le verrouillage à usage unique, de préférence par moulage par injection ou par moulage par injection de précision.

8. Dispositif d'abrasion selon l'une des revendications précédentes, **caractérisé en ce que** les saillies ont une forme tétraédrique, pyramidale ou au moins sensiblement conique.

9. Dispositif d'abrasion selon l'une des revendications précédentes, **caractérisé en ce que** la hauteur des saillies est comprise entre 50 et 1500 µm.

10. Dispositif d'abrasion selon l'une des revendications précédentes, **caractérisé en ce que** la rotation de l'unité d'abrasion (2) s'effectue à une fréquence de 50 à 200 Hz.

11. Dispositif d'abrasion selon l'une des revendications précédentes, **caractérisé en ce que** la fréquence de rotation de l'unité d'abrasion (2) est réglable, de préférence en continu.

12. Dispositif d'abrasion selon l'une des revendications 3 à 12, **caractérisé en ce que** la surface d'abrasion (5) est de forme circulaire et présente un diamètre de surface compris entre 2 et 100 mm.

13. Dispositif d'abrasion selon l'une des revendications 3 à 12, **caractérisé en ce que** la surface d'abrasion (5) est triangulaire ou carrée.

14. Dispositif d'abrasion selon l'une des revendications précédentes, **caractérisé en ce que** le manchon de protection (11) est amovible.
